# EUROPEAN PATENT APPLICATION

(11) **EP 3 202 338 A1**
(43) Date of publication of application: **09.08.2017**
(21) Application number: 16305143.6
(22) Date of filing: 05.02.2016
(51) Int. Cl.: A61B 17/11

(54) **ANASTOMOTIC CONNECTOR**

(71) Applicant: CHU de Nice, 06000 Nice (FR)
(72) Inventor: ROSELLO, Olivier, 06000 Nice (FR)
(74) Representative: Osha Liang

(57) **Abstract**

An anastomotic connector comprising: a connector body 1 having a first end 10 engageable into a first vessel V1, the first end 10 being provided with teeth 13 on its outer surface, and a first locking sleeve engageable around the first end 10, the first locking sleeve surrounding the teeth 13 in a locked position, in order to impede relative movement between the first vessel V1 and the connector body 1. The first locking sleeve is provided with longitudinal ribs on its inner surface, the ribs being spaced from each other in a circumferential direction of the first locking sleeve.

## Description

### TECHNICAL FIELD

The present disclosure relates generally to an anastomotic connector, that is a device for interconnecting separate or severed tubular hollow structures to form a continuous channel. In particular, it relates to a vascular anastomotic connector.

### BACKGROUND

A vascular anastomosis is a connection between two blood vessels. The connection may be established between two vessel ends (then, it is referred to an end-to-end anastomosis), or between the end of one vessel and the side of another vessel (then, it is referred to an end-to-side anastomosis). The vessels may be natural or prosthetic.

Vascular anastomoses are still today widely carried out by suturing the vessels with a non-resorbable surgical thread or by using surgical staples. Although globally satisfactory, these techniques take time and, if not performed correctly, may cause blood loss or stenosis in the vessel-connection area.

As an alternative to sutures and surgical staples, anastomotic connectors may be used.

Examples of anastomotic connectors are described, for instance, in patent document FR 2683141. These connectors have two opposite ends to be inserted, respectively, into vessel ends. The connector end may have barbs or circumferential ridges on its external surface, for preventing the vessel from sliding out of the connector end. An external clamp is provided around the vessel portion overlying the connector end to hold the vessel in place on the connector.

While conventional anastomotic connectors have proven effective, because of the constant micro-motions of the vessels (due to the oscillating blood pressure), blood leakage may happen in the vessel-connection area and the vessel may slide out of the connector end, even when an external clamp is provided. Further, if the external clamp is clamped too tight around the vessel, there is a risk of damaging the vessel portion overlying the connector and, in particular, of cutting circumferentially the vessel. If the vessel is damaged by the external clamp, this may, in turn, be detrimental to the leak-tightness of the connection or to the fixing of the vessel.

Accordingly, there exists a need for a new kind of anastomotic connector addressing the aforementioned problems, or at least providing the practitioners with a useful alternative to the conventional connectors.

### GENERAL PRESENTATION

According to one aspect of the present disclosure, there is provided an anastomotic connector comprising: a connector body having a first end engageable into a first vessel, and a first locking sleeve engageable around the first end. The first end of the connector body is provided with teeth on its outer surface, and the first locking sleeve surrounds the teeth in a locked position, in order to impede relative movement between the first vessel and the connector body. The first locking sleeve is provided with longitudinal ribs on its inner surface. The ribs are spaced from each other in a circumferential direction of the sleeve.

When the first locking sleeve surrounds the teeth, the first vessel is trapped between the first locking sleeve and the connector body and the ribs push the vessel towards the teeth. Thus, the risk that the vessel disengages from the teeth is limited and the leak-tightness between the connector body and the vessel is improved.

Moreover, the risk of damaging the first vessel is limited due to the orientation and position of the ribs. In particular, as compared to a circumferential ridge or analog, the risk of cutting or constricting circumferentially the first vessel is reduced. This is a beneficial for the fixing of the first vessel and for the growth of tissue cells around this vessel.

The first locking sleeve has a main axis, or central line, extending between its two main opposite openings and passing through the center of each cross-section of the sleeve. In the frame of the present disclosure, the length of the sleeve is the dimension along the main axis of the sleeve. The ribs are said to be longitudinal because they extend substantially in a longitudinal direction of the sleeve. A circumferential direction follows the circumference of a cross-section of the sleeve. A radial direction is a direction perpendicular to the main axis. The "inner" and "outer" parts or surfaces of an element are considered in relation to the radial direction. Thus, for instance, in case of a sleeve having a cylindrical shape, the main axis is the revolution axis. A longitudinal direction is parallel to the main axis, a circumferential direction is circular around the main axis and a radial direction is perpendicular to the main axis. The same applies for the connector body. Finally, the words "distal" and "proximal" are considered in relation to the center of the connector and lengthwise; one element (or one part of element) is proximal to another element (or another part of the element) when it is closer to the center of the connector than the other element (or the other part of the element), lengthwise.

The teeth provided on the outer surface of the connector end may have various shapes: they may have various thicknesses, various heights, be more or less sharp, etc. For instance, the teeth may be barbs, spikes, small peaks, etc. Also, the teeth may be in various positions. In particular, they may be arranged on the outer surface in at least two circumferential rows spaced from each other. This allows the vessel to be better held on the connector end. The teeth may be all the same or different from each other, depending on their position.

In certain embodiments, there are two rows of teeth spaced from each other by a first distance, while the longitudinal ribs have a length greater than or equal to the first distance and extends over the rows of teeth in the locked position. This allows the vessel to be better held on the connector end.

In certain embodiments, the first locking sleeve is provided with through holes or slots extending from the inner surface to the outer surface of the locking sleeve. Thus, after implantation, tissue cells may grow inside the holes or slots, from the vessel to the outside of the locking sleeve.

In certain embodiments, the first locking sleeve and the first end are provided with a locking system that maintains the first locking sleeve in a fixed position relative to the first end, this fixed position corresponding to said locked position. The locking system may be such that, for moving the first locking sleeve to the locked position, the first locking sleeve has first to be moved lengthwise onto to the first end and then rotated around the first end. When the locking sleeve is turned, the ribs may pass over the teeth and push the vessel onto the teeth, thereby helping the teeth to penetrate into the vessel thickness. The rotation of the sleeve may, however, be limited to avoid damaging the vessel. For instance, the rotation may not exceed a quarter or one-eighth of a turn.

The connector body and the first locking sleeve may be spaced from each other by a radial gap when the first locking sleeve is in the locked position. The height of the teeth and/or the height of the ribs may be both lower than the radial gap. If not, the teeth and/or the ribs are deformable. In addition, the sum of the tooth height and the rib height may be lower than the radial gap. If not, the ribs are sufficiently deformable to pass over the teeth when the first locking sleeve is rotated around the first end.

In certain embodiments, when the first locking sleeve is in the locked position, the ribs are fitted in between the teeth. In particular, this means that the ribs are fitted in between the teeth after the first locking sleeve has been rotated to its locked position. Thus, the pressure applied on the vessel is limited (as compared to a situation where the ribs are in front of the teeth in the locked position), which decreases the risk of damaging the vessel in the long term.

In certain embodiments, the connector body has a second end similar to the first end and opposed thereto, the second end being engageable into a second vessel. Then, the connector is provided with a second locking sleeve, similar to the first locking sleeve, engageable around the second end in order to impede relative movement between the second vessel and the connector body.

According to another aspect of the present disclosure, there is provided a method for anastomosis comprising the steps of: providing an anastomotic connector as described above, engaging the first end of the connector body into the first vessel, and moving the first locking sleeve to its locked position in order to impede relative movement between the connector and the first vessel. Such a method may be used for establishing a connection between two vessel ends, i.e. for an end-to-end anastomosis, or for establishing a connection between the end of one vessel and the side of another vessel, i.e. for an end-to-side anastomosis. For an end-to-end anastomosis, a connector body with two similar opposite ends may be used and the method may further comprise: engaging the second end of the connector body into the second vessel, and moving the second locking sleeve to its locked position in order to impede relative movement between the connector and the second vessel.

Before engaging the first end of the connector body into the first vessel, the first locking sleeve may be slipped on the first vessel. Similarly, before engaging the second end of the connector body into the second vessel, the second locking sleeve may be slipped on the second vessel.

Such a method has the advantages derived from using an anastomotic connector according to the present disclosure.

Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same or like parts throughout the different views. The drawings are not necessarily to scale, emphasis instead being placed upon illustrating the principles of the invention.
FIG 1 is a perspective view of an example of an anastomotic connector including a connector body and two locking sleeves, respectively engaged around the two opposite ends of the connector body.
FIG 2 is a side view of the connector body of FIG 1, following arrow II.
FIG 3 is a front view of the connector body of FIG 1, following arrow III.
FIG 4 is a perspective front view of one of the locking sleeves of FIG 1.
FIG 5 is a perspective back view of one of the locking sleeves of FIG 1.

### DETAILED DESCRIPTION

In the following detailed description, it is referred to the accompanying drawings showing an example of anastomotic connector. It is intended that this example be considered as illustrative only, the invention not being limited to this example.

To avoid unnecessary details for practicing the invention, the description may omit certain information already known to those skilled in the art.

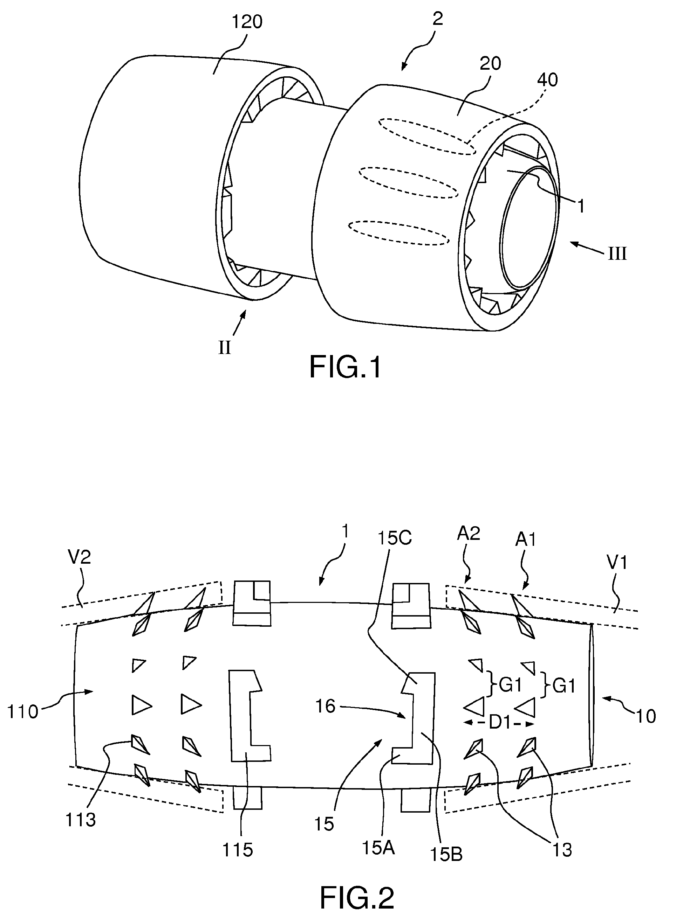

With reference to the figures, the reference number 2 generally designates an anastomotic connector, that is a connector for establishing a connection between two blood vessels V1, V2, shown in dotted line in FIG 2. In this example, the connector 2 is an end-to-end anastomotic connector, which means that it establishes a connection between a free end of a first vessel V1 and a free end of a second vessel V2. The connector 2 comprises a connector body 1 with two opposite ends 10 and 110. The first and second ends 10, 110 are adapted for being engaged into the first and second vessels V1, V2, respectively. To make this engagement easier, the first and second ends 10, 110 may be tapered, as illustrated in FIG 2. The first and second ends 10, 110 are provided with teeth 13, 113 on their outer surface. These teeth 13 are adapted to penetrate into the vessel inner wall for holding the vessels V1, V2 in position on the first and second ends 10, 110.

The connector 2 further comprises two locking sleeves 20, 120 which are adapted for being engaged around the first and second ends 10, 110, respectively. In FIG 1, the first and second locking sleeves 20, 120 are shown in their locked position. In this locked position, the locking sleeves 20, 120 overly the teeth 13, 113, respectively.

In the illustrated example, the two opposite ends of the connector 1 are similar. In particular, the first and second ends 10, 110 of the connector body 2 are similar, and the locking sleeves 20, 120 are similar. Therefore, for the sake of conciseness, only the first connector end 10 with the locking sleeve 20 will be described below.

In order to maintain the first locking sleeve 20 in its locked position on the first end 10, the locking sleeve 20 and the first end 10 are provided with hooking parts 15, 25 cooperating with each other. In the illustrated example, the hooking parts 15 protrude on the outer surface of the first end 10 and are proximal to the teeth 13 (i.e. closer to the center of the connector body 1 than the teeth 13, lenghtwise). The hooking parts 15 are circumferentially spaced from each other. At least one hooking part 15 (each hooking part 15 in this example) comprises a base 15B and two branches 15A, 15C extending from the base 15B in the proximal direction, thereby forming a "U" with a notch 16 defined between the base 15B and the branches 15A, 15C. The notch 16 is on the proximal side of the hooking part 15. The hooking parts 25 of the locking sleeve 20 are tabs protruding from the inner surface and located at the proximal end of sleeve 20. The hooking parts 25 are circumferentially spaced from each other, and adapted to fit in the notches 16. For moving the hooking parts 25 into the notches 16, the first locking sleeve 20 has first to be slipped onto to the first end 10, so that each hooking part 25 passes between and goes beyond two adjacent hooking parts 15. Then the locking sleeve 20 is rotated around the first end 10, so that the hooking part 25 passes over the branch 15C. To facilitate this step, the end of the branch 15C may be sloped, as illustrated in FIG 2. Then, the hooking part 25 engages into the notches 16 and the hooking part 25 is released. When the hooking parts 25 are engaged in the notches 16, the first locking sleeve is held in a fixed position relative to the first end 10, corresponding to the locked position.

Going back to the teeth 13, they are arranged on the outer surface in at least two circumferential rows A1, A2 spaced from each other, lengthwise, by a first distance D1. In each row A1, A2, the teeth 13 are separated from each other by circumferential gaps G1. Also, the teeth 13 of the rows A1, A2 are aligned in a longitudinal direction of the connector body 2, so that gaps G1 are aligned in a longitudinal direction.

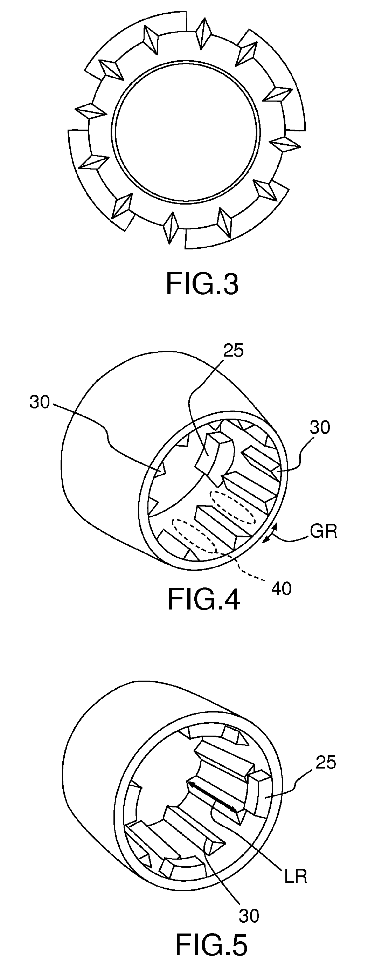

As shown in FIGS 4 and 5, the sleeve 20 is provided with longitudinal ribs 30 on its inner surface. The ribs 30 have a length LR greater than or equal to the first distance D1, and are spaced from each other in a circumferential direction of the first locking sleeve by a circumferential gap GR. When the first locking sleeve is held in the fixed position, each rib 30 extends longitudinally over the tooth rows A1, A2, while being fitted circumferentially in between adjacent teeth 13. The ribs 30 may be tapered towards their ridge. In particular, the ribs 30 may have a thin ridge line and their cross-section is substantially triangular.

Finally, the sleeve 20 may be provided with through holes or slots 40 (shown in dotted lines in FIGS 1 and 4) extending from the inner surface to the outer surface of the sleeve, for promoting tissue growth through the sleeve 20 after implantation. The slots 40 may extend longitudinally between the longitudinal ribs 30.

While only one selected embodiment has been chosen to illustrate the present invention, it will be apparent to those skilled in the art from this disclosure that various changes and modifications can be made herein without departing from the scope of the invention as defined in the appended claims. For example, the size, shape, location or orientation of the various components can be changed as needed and/or desired. Further, it is not necessary for all advantages to be present in a particular embodiment at the same time.

## Claims

1. An anastomotic connector comprising:
a connector body (1) having a first end (10) engageable into a first vessel (V1), the first end being provided with teeth (13) on its outer surface,
a first locking sleeve (20) engageable around the first end (10), the first locking sleeve (20) surrounding the teeth (13) in a locked position, in order to impede relative movement between the first vessel (V1) and the connector body (1),
wherein the first locking sleeve (20) is provided with longitudinal ribs (30) on its inner surface, the ribs being spaced from each other in a circumferential direction of the first locking sleeve (20).

2. The anastomotic connector of claim 1, wherein the teeth (13) are arranged on the outer surface in at least two circumferential rows (A1, A2) spaced from each other.

3. The anastomotic connector of claims 1 and 2, wherein the two rows of teeth (13) are spaced from each other by a first distance (D1), wherein the ribs (30) have a length (LR) greater than or equal to the first distance (D1), and wherein the ribs (30) extends over the rows of teeth (A1, A2) in the locked position.

4. The anastomotic connector of any of claims 1 to 3, wherein the first locking sleeve (20) is provided with through holes or slots (40) extending from the inner surface to the outer surface of the first locking sleeve (20).

5. The anastomotic connector of any of claims 1 to 4, wherein the first locking sleeve (20) and the first end (10) are provided with a locking system that maintains the first locking sleeve (20) in a fixed position relative to the first end (10), corresponding to said locked position.

6. The anastomotic connector of claim 5, wherein the locking system is such that, for moving the first locking sleeve (20) to the locked position, the first locking sleeve (20) has first to be moved lengthwise onto to the first end (10) and then rotated around the first end (10).

7. The anastomotic connector of any of claims 1 to 6, wherein the ribs (30) are sufficiently deformable to pass over the teeth (13) when the first locking sleeve (20) is rotated around the first end (10).

8. The anastomotic connector of any of claims 1 to 7, wherein the locking system comprises hooking parts (15, 25) provided on both the first end (10) and the first locking sleeve (20), and cooperating with each other for maintaining the first locking sleeve (20) in the locked position.

9. The anastomotic connector of any of claims 1 to 8, wherein the ribs (30) are fitted in between the teeth (13) when the first locking sleeve (20) is in the locked position.

10. The anastomotic connector of any of claims 1 to 9, wherein the connector body (1) has a second end (110) similar to the first end (10) and opposed thereto, the second end (110) being engageable into a second vessel (V2), the connector comprising a second locking sleeve (120) similar to the first locking sleeve (20) and engageable around the second end (110) in order to impede relative movement between the second vessel (V2) and the connector body (1).

11. A method for anastomosis, comprising the steps of:
providing an anastomotic connector according to any one of the preceding claims,
engaging the first end (10) of the connector body (1) into a first vessel (V1), and
moving the first locking sleeve (20) to its locked position, in order to impede relative movement between the connector and the first vessel (V1).

12. The method of claim 11, further comprising the steps of:
engaging the second end (110) of the connector body (1) according to claim 10 into a second vessel (V2), and
moving the second locking sleeve (120) to its locked position, in order to impede relative movement between the connector and the second vessel (V2).
